Europäisches Patentamt

(19) European Patent Office

Office européen des brevets

(11) Publication number : **0 293 372 B1**

(12) # EUROPEAN PATENT SPECIFICATION

(45) Date of publication of patent specification :
19.06.91 Bulletin 91/25

(51) Int. Cl.⁵ : **A61B 5/11**

(21) Application number : **87900723.5**

(22) Date of filing : **19.12.86**

(86) International application number :
**PCT/EP86/00767**

(87) International publication number :
**WO 88/04536 30.06.88 Gazette 88/14**

(54) NON-INVASIVE QUANTITATIVE KNEE JOINT IN VIVO INSTABILITY ANALYZER.

The file contains technical information submitted after the application was filed and not included in this specification

(43) Date of publication of application :
07.12.88 Bulletin 88/49

(45) Publication of the grant of the patent :
19.06.91 Bulletin 91/25

(84) Designated Contracting States :
DE FR GB

(56) References cited :
EP-A- 204 639
US-H-10 060 2
The Journal of Bone and Joint Surgery, volume 60A, no. 5, July 1978, The Journal of Bone and Joint Surgery, Inc., K.L. Markolf et al.:" In vivo knee stability"

(56) References cited :
Medical & Biological Engineering & Computing, volume 15, no. 5, September 1977, (Stevenage, GB), P.J. Lowe et al.:" Knee analyser: An objective method of evaluating medio-lateral stability in the knee"
Journal of Applied Mechanics, volume 43, no. 1, March 1976, (New York, US), G.I. Zahalak et al.:" Partially activated human skeletal muscle: An experimental investigation of force, velocity, and EMG"

(73) Proprietor : LUSUARDI, Werther
Kreuzbühlstrasse 8
CH-8008 Zürich (CH)
Proprietor : Al-Turaiki, Mohammed H.S.
P.O. Box 65
Al-Zulfi (SA)

(72) Inventor : AL-TURAIKI, Mohammed H.S.
P.O. Box 65
Al-Zulfi (SA)

(74) Representative : Lusuardi, Werther Giovanni
Dr. Lusuardi AG Kreuzbühlstrasse 8
CH-8008 Zürich (CH)

## Description

This invention relates to a non-invasive quantitative knee joint in vivo instability analyzer with means for fixing the thigh and femoral condyles, means for fixing the foot and malleoli and means to cater for various angles of knee and hip flexion.

Of all joints in the human body the knee joint is the most vulnerable to traumatic injury. These injuries arise from a wide range of causes, including vehicular accidents, but particularly from a wide spectrum of sporting and athletic activities. In order to reduce the number of such injuries and ensure successful joint reconstruction, accurate methods are required for diagnosing both chronic and early ligamentous instabilities which, if not treated, may render the knee significantly more susceptible to injury.

Knee instability is defined as an abnormal rotational and/or translational movement of the tibial plateaux in relation to the femoral condyles, occurring about one or more axes or in one or more planes of motion and resulting in a functional deficit. Knee stability is provided by the complex interaction of a multitude of factors, including ligaments and other soft-tissue restraints, condylar geometry, active muscular control, and tibio-femoral contact forces generated during weight-bearing activities.

Instability is caused when one or more of the structures which normally contribute to knee stability are injured. The magnitude of the instability induced by such a disruption is, however, difficult to quantify.

There are four types of instability which involve no rotation of the tibia with respect to the femur :
  (a) Valgus instability
  (b) Varus instability
  (c) Posterior instability
  (d) Anterior instability.

There are also four types of simple rotatory instability :
  (a) Anteromedial rotatory instability
  (b) Anterolateral rotatory instability
  (c) Posterolateral rotatory instability
  (d) Posteromedial rotatory instability

The rotatory instabilities of type b/c, a/b, and a/b/c may be combined.

At present, the clinical assessment of knee joint instability is carried out subjectively by setting the hip and knee at roughly the required angles, applying unknown forces to the lower limbs, and observing the resulting displacements. The success of such methods depends to a large extent on the judgement and experience of the examining physician who attempts in the examination to isolate only the passive restraints provided by the ligaments. This limited and subjective evaluation of the joint explains the frequent disparity between the results of a stability examination and the observed in-vivo joint function. Clearly, reliable non-invasive quantitative objective techniques for the assessment of knee joint instability are needed if over or under treatment is to be avoided. Further, if the progress of patients is to be monitored over a period of time, reliable comparisons of knee stabilities on different occasions, or between different individuals, are required.

The need to objectively assess knee stability has been recognised by many workers, and a number of different devices have been devised and tested with varying degrees of success. Most of these devices were designed to exclusively assess one, two, or in an extremely few instances [e.g. Markolf et al., Orthopaedic Research Society, No. 110 (1975) ; No. T176 (1976) and J. Bone & Int. Surg., 58A, No. 5 (July, 1976) ; 60A, 664-674 (July, 1978)] three types of instability ; the anterior-posterior drawer sign, valgus-varus rotation, and tibial torsion.

This necessarily limited the amount of information which could be made available to the orthopaedic surgeon caring for the patient. In a few cases, the levels of force applied during the tests were specified and real-time responses could be obtained. Nevertheless, the precise manner in which loads were applied was not defined, thus reducing the validity of the measurement obtained. Furthermore, in all the techniques described there has been no mention of any objective means employed to ensure that the stability measured arose from the passive restraints and was not due to protection of the knee joint by its musculature. Muscle forces, neuromuscular coordination, and the other factors all determine whether there is a functional stability or otherwise.

From Siu et al. [Biomechanics Symposium, ASME ; New York, 193-196(1981)] a computerised knee analyser is known to assess mediolateral stability in the human knee joint in vivo. The knee is thereby subjected to a maximum bending moment of 20 NM through a motorised loading mechanism.

From Shoemaker et al. [J. Bone & Jnt. Surg., 64-A, 208-216 (Feb.,1982)] an instrumented clinical knee testing apparatus is known capable of assessing tibial axial rotational instability. Torque and resulting angular displacement are recorded with the aid of electrically operated transducers. The equipment used allows different angles of knee and hip flexion.

An apparatus is known from US T100,602 ROLEY suitable for measuring knee laxity and stiffness. This prior art device allows the application of an axial torque to the knee-joint by moving a lever. It is not possible with this known device, however, to apply incremental loads and torques to the knee joint via load and torque transducers.

It is also known from "The Journal of bone and joint surgery", Volume 60A, Number 5, July 1978, K.L. Markolf et al. : "In vivo knee stability" page 664-674 to use transducers to record anterior-posterior tibial force versus displacement and varus-valgus moment versus angulation during manual manipulation of the knee joint.

The known knee joint analysers, in particular the apparatus according to US-T100,602 ROLEY have failed, however, to satisfactorily produce a reliable system for the non-invasive quantitative assessment of human knee instability in vivo. They can be criticised on four main points :

(1) Recordings of muscular activity are not possible

(2) Failure to define the mechanical parameters of the tests carried out with the analyser (factors such as the rate of loading may significantly affect the results obtained)

(3) Failure of comprehensive assessment of the knee joint

(4) Impossibility to measure dynamic stability resulting from muscular contraction and weight-bearing.

## Summary of the Invention

Accordingly, the present invention aims to provide a device which can be used reliably to quantify knee instabilities by performing four non-invasive stability tests : (1) anterior-posterior drawer sign ; (2) valgus-varus rotation ; (3) tibial torsion ; and (4) médial-lateral drawer sign.

Another object of the invention is to provide a device capable of assessing the effects of muscular contraction and weight-bearing on the knee joint stability.

Still another object of the invention is to provide a device capable of specifying the maximum loads and rate of loading.

Yet another object of the invention is to provide a device capable of assessing the stabilizing effect on the joint of the tibiofemoral contact force, thus giving an indication of the integrity of the joint congruency.

Yet another object of the invention is to provide a device capable of measuring the torques generated by muscle contraction.

Other objects will, in part, be obvious and will, in part, appear hereinafter.

The invention accordingly comprises the several steps and the relation of one or more steps with respect to each of the others, and the apparatus embodying the features of construction, combinations of elements and arrangement of parts which are adapted to effect such steps, all as exemplified in the following detailed description.

Briefly, any apparatus is aimed at allowing the sage and accurate measurement of knee stability in various test planes, incorporating the following functions :

(1) comfortable seating of the patient

(2) provision for securely clamping the tigh and femoral condyles

(3) provision for securely clamping the foot and malleoli

(4) adjustability for different leg lengths

(5) adjustability for varying degrees of knee and hip flexion

(6) loading the tibia axially with continuous measurement of applied load

(7) loading the tibia anteriorly or posteriorly with continuous measurement of load applied and resulting anterior or posterior displacement

(8) loading the tibia medially or laterally with continuous measurement of load applied and resulting medial or lateral displacement

(9) applying valgus or varus moments to the knee with continuous measurements of applied moment and resulting valgus or varus rotation

(10) applying external or internal axial torques to the tibia with continuous measurement of torques applied and external or internal tibial rotation

(11) measurement of activity of muscles around the knee joint

(12) monitoring continuously EMG signals from musculature.

## Brief Description of the Drawings

For a fuller understanding of the nature and objects of the invention, reference should be made to the following detailed description, taken in connection with the accompanying drawings, in which :

FIG. 1 is a perspective view of the test knee analyser ;

FIG. 2 is an exploded view of the central pivot and locking mechanism ;

FIG. 3 is perspective view showing the transducer system of the knee analyzer according to the invention ; and

FIG. 4 is schematic representation of the measurement and recording system of the knee analyzer according to the invention.

## Description of the Preferred Embodiments

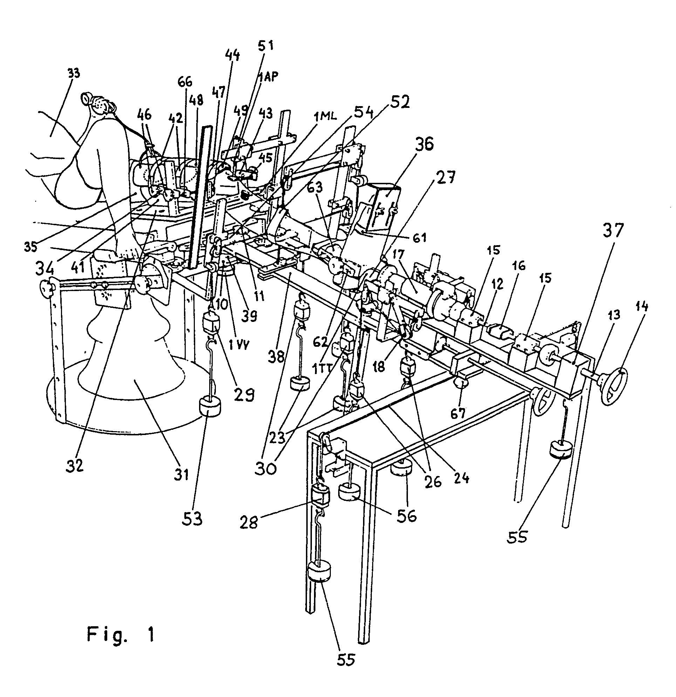

Referring now to FIG. 1 of the drawings, the apparatus according to the invention consists essentially of a dental chair 31 modified by the addition of a rigid frame 32 to which fixtures can be securely attached. The chair 31, being heavy, provides a stable testing base, while its adjustability allows subjects of various sizes to be accomodated with knees flexed throughout the range of 0° to 90° flexion. Additionally, subjects can be placed supine, to relax the hamstrings, by adjusting the movable chairback 33.

The chair 31 comprises a thigh clamp 34 for fixing the tigh 35 and the femoral condyles and a plate 36 to which is strapped the patient's foot.

The entire clamping system consists of two distinct parts : one for the thigh and femoral condyles, and the other for the foot and malleoli.

The thigh 35 is supported posteriorly by a curved thigh plate 41 which has been stiffenend sufficiently for it to appear rigid under the loads to which it is subjected. To fix the tigh to the thigh plate 41, two velcro straps 42 are used. The design adopted consists of a lateral and medial rectangular aluminium block 43 with circular recess to which asand-filled leather pouch 44 is glued. A screw 45 is fixed to the centre of the block 43, enabling it to be adjusted. Said screw 45 passes through a hole in an adjustable clamp fixed to the metal framework 32 surrounding the knee. The pouches 44 can be advanced to clamp the condyles and locked in position using the two lock nuts 66. Two straps are fixed to the pouches 44 ; one strap (not visible in the figure) to support the popliteal region from below, and the other strap 47 to provide a third point of fixation of a tourniquet 48 to the thigh clamp 34. Between the tourniquet 48 and the patella is placed a sand-filled fabric pouch 49 which conforms to the shape of the patellar region and provides a pain-free firm fixation. The pneumatic three-bladder tourniquet 48 forms a half-cuff around the thigh, and when inflated presses the thigh firmly against the thigh-plate 41, and the patella against the popliteal strap.

The foot rest against a plate 36, of which the length can be adjusted, with the heel supported on an adjustable pad made of aluminium covered with plastozote. The foot is strapped to said plate 36 by a velcro strap 61 which passes around the metatarsal region. To said foot plate 36 are fixed two metal strips 62 which provide fixation for the pads 63 which are used to clamp the malleoli. Said pads 63 are similar to the pouches 44 used to clamp the femoral condyles. The malleoli are further fixed via a velcro strap surrounding said metal strips 62.

The apparatus according to the invention has two separate loading systems. Firstly, the axial loading system used to simulate load bearing on the limb and secondly, the loading system used to displace the tibia relative to the femur in the actual stability test.

The axial load and torque loading mechanism 37 comprises the foot plate 36 which is mounted on a shatf 12 which runs parallel to the axis of the limb and is butted onto the end of a screw 13 which terminates distally in a small handwheel 14. When said handwheel 14 is rotated it advances the screw 13, thus pushing the foot-plate mounting shaft 12 proximally, and hence applies an axial load to the limb. Said footplate shaft 12 is supported in a number of bearings 15 and is interrupted by an axial load transducer 16 and a torque transducer 17. It also carries a pulley 18 by means of which axial torques can be applied to the limb.

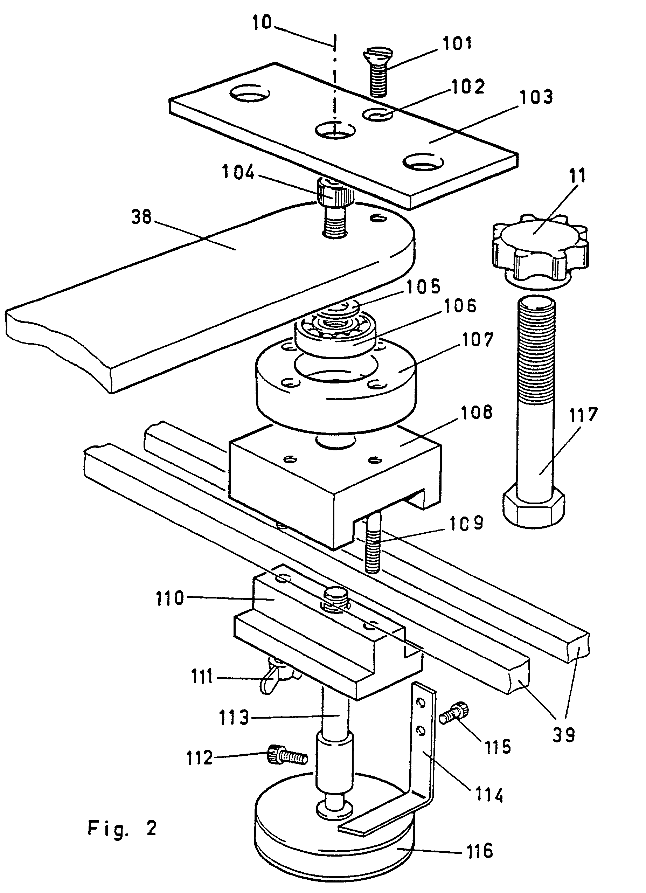

The footplate 36 is connected to said loading mechanism 37 via load, torque and angular displacement transducers (4 and 6). The position of the loading mechanism 37 can be adjusted to suit the patient's leg length by sliding it along a guide bar 38 which in turn is attached to a cross-arm 39 at a central-pivot 10 along which it can rotate (the central pivot and locking mechanism is represent in FIG. 2 in more detail). This cross-arm 39, which is rigidly attached to the chair 31 provides adjustement in the vertical plane to cater for various angles of knee flexion. For the measurement of linear displacement in the knee, adjustable arms fitted with linear displacement transducers are provided which are attached to the cross arm 39. For the application of loads to the knee in various planes, adjustable arms fitted with pullies are provided. The system of pullies and weights is operated manually.

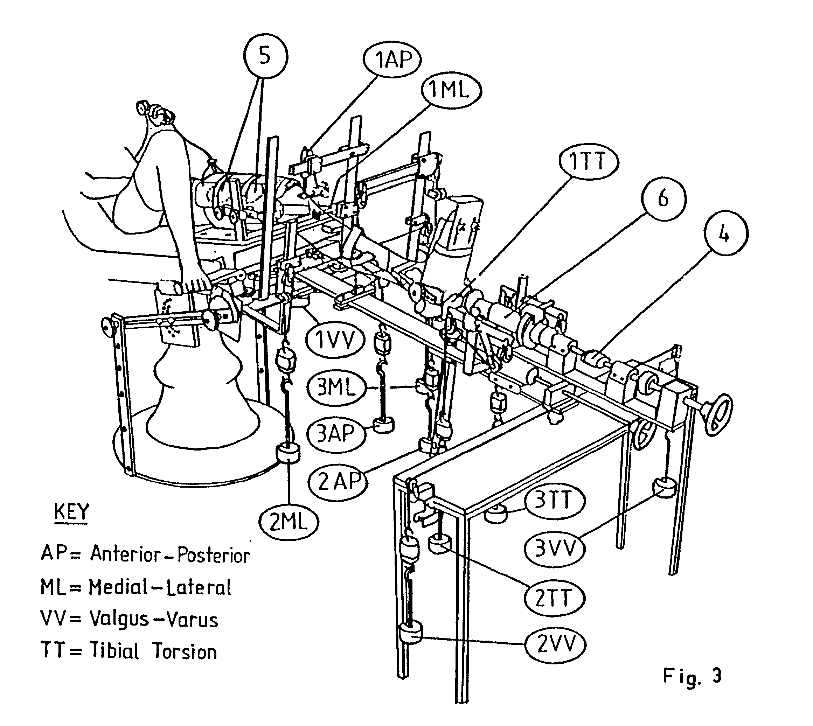

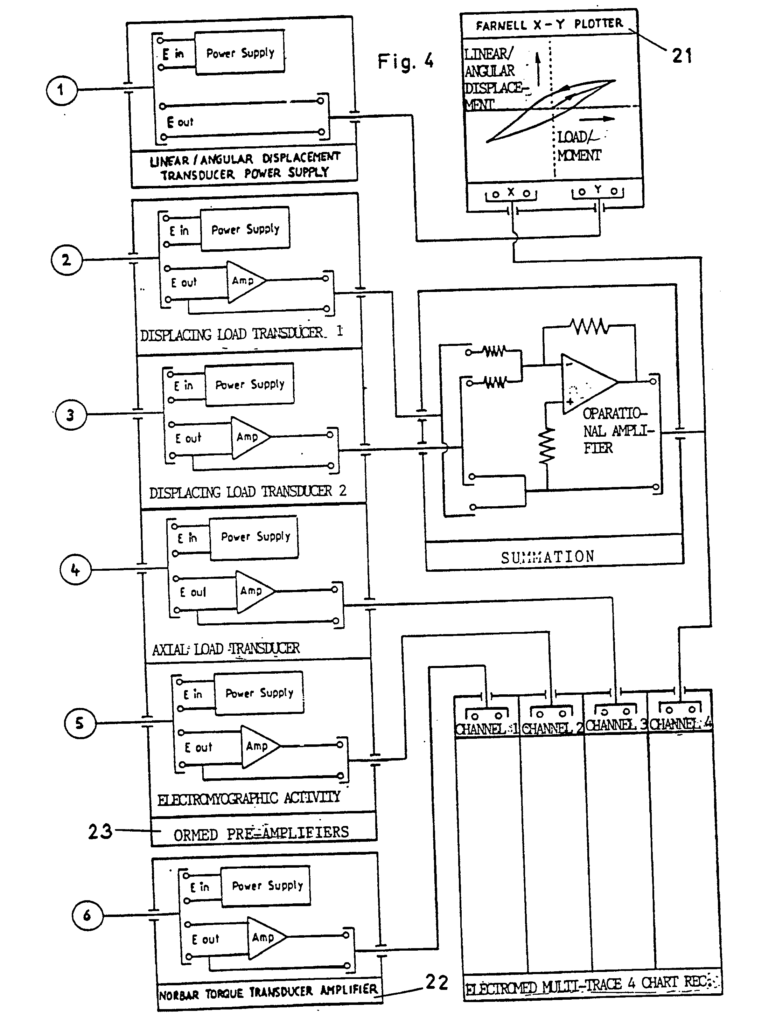

The test loading system consists of a dead loading system. Cords 24 attached to fittings applied to the lower limb are passed over pullies 18 mounted on adjustable arms and bearing weights 23 added to hangers tied to their other ends. Interposed in the cords 24 are transducers for measuring the applied loads (Figs. 3 and 4).

Linear displacement is measured by linear motion conductive plastic potentiometers 1AP and 1ML. These produce an electrical signal directly proportional to the linear mechanical movement of the shaft ; conductive plastic resistance elements provide virtually infinite resolution and long life expectancy. The potentiometric transducer provides a full scale output equal to the applied voltage so that there is no need for an amplifier or other circuitry. The transducers should preferably have a nominal mechanical stroke of 50 mm, an electrical stroke of 50 ± 0,5 mm and an independent linearty of ± 0,3%.

Angular displacement is measured by rotary motion conductive plastic continuous potentiometers 1VV and 1TT which provide an electrical signal directly proportional to the rotary mechanical movement of the shaft. The transducer has preferably a mechanical traval of 360°, an electrical travel of 350 ± 5°, and an independent linearity of ±3%.

Forces applied to the knee are measured by load transducers 2, 3 and 4 with foil strain gauges bonded onto a Z beam type body. The axial load transducer 4 has a capacity range of preferably 0-2,5 KN, whilst the displacing load transducers 2 and 3 have a capacity range of 0-0,5 KN. All load transducers 2, 3 and 4 have an infinite resolution, and a terminal non-linearity of < 0,05%. Axial torque is measured by a standard torque transducer 6 which comprises a solid stainless steel shaft, onto which strain gauges are bonded. The torque transducer 6 has a capacity range of 0-500 Nm, and a non-linearity of ± 0,25%.

The displacement transducers 1 give large voltage outputs in the range of 0-5 V and can therefore be directly connected to the recording instrument 21 without the need for amplification.

The load transducers 2, 3 and 4 require an excitation voltage of 10 V and have a normal sensitivity of 3 mv/V. Therefore their outputs have to be amplified using a commercially available transducer pre-amplifier 23. The torque transducer 6 requires an excitation voltage of 2,4 VDC and should have a sensitivity of 3 mv/V. The output is amplified by a matching amplifier 22.

The knee response curve is obtained by connecting the outputs from the test load and displacement transducers to the x and y channel of a X-Y plotter (Figure 4). The load signal can also be fed for synchronization purposes, to a four-channel chart recorder to which the axial load and EMG outputs are also connected.

## Claims

1. Non-invasive quantitative knee joint in vivo instability analyser comprising,

means (31, 32) for positioning knee and hip flexed at various angles,

means (31, 32, 34, 41, 42, 44) for fixing the thigh and femoral condyles,

means (36) for fixing the foot and malleoli comprising a foot plate being mounted on a rotatable shaft (12) which runs parallel to the axis of the limb, said shaft (12) being supported by a number of bearings (15),

means (23) for loading the tibia anteriorly or posteriorly and means (30) for continuously measuring the load applied and means (51, 1AP) for measuring the resulting anterior or posterior displacements,

means (53) for loading the tibia medially or laterally and means (29) for continuously measuring the load applied and means (1ML) for measuring the resulting medial or lateral displacements,

means (55, 24) for applying valgus or varus moments to the knee and means (28) for continuously measuring the moments applied and means (1VV) for measuring the resulting valgus or varus rotation,

means (56) for applying external or internal axial torques to the tibia and means (26) for measuring the applied torques and means (1TT) for measuring external or internal tibial rotation,

whereby the loads are applied via a system of pulleys (18), cords (24) and weights (23),

characterised in that

transducers (26, 28, 29, 30) are interposed in the cords (24),

and in that said shaft (12) is butted onto the end of an advancing screw (13) for pushing said foot plate (36) proximally for applying an axial load to the limb, and in that said shaft is interrupted by an axial load transducer (16) and a torque transducer (17).

2. Knee joint analyzer according to claim 1, wherein means are provided for monitoring continuously EMG signals from the knee musculature.

## Revendications

1. Analyseur quantitatif non-invasif in vivo de l'instabilité de l'articulation du genou avec

moyens (31, 32) pour le positionnement du genou et de la hanche fléchi sous différents angles,

moyens (31, 32, 34, 41, 42, 44) pour la fixation du fémur et des condyles du fémur,

moyens (36) pour la fixation du pied et des malléoles, comprenant un support de pied monté sur un arbre rotatif (12) disposé parallèlement à l'axe du membre, cet arbre (12) étant soutenu par un nombre de supports (15),

moyens (23) pour charger le tibia antérieurement ou postérieurement et moyens (30) pour mesurer continuellement la charge appliquée et moyens (51, 1AP) pour mesurer les déplacements résultants, antérieures ou postérieures,

moyens (53) pour charger le tibia médialement ou latéralement et moyens (29) pour mesurer continuellement la charge appliquée et moyens (1ML) pour mesurer les déplacements résultants, médiales ou latérales,

moyens (55, 24) pour appliquer un moment en valgus ou varus au genou et moyens (28) pour mesurer continuellement les

moments appliqués et moyens (1VV) pour mesurer la rotation résultante en valgus ou varus,

moyens (56) pour appliquer des moments de torsion externes ou internes au tibia et moyens (26) pour mesurer continuellement les moments de torsion appliqués et moyens (1TT) pour mesurer la rotation tibiale résultante, externe ou interne,

les charges étant appliquées au moyen d'un système de poulies (18), cordes (24) et poids (23),

caractérisé en ce que

transducteurs (26, 28, 29, 30) sont disposés entre les cordes (24),

et en ce que l'arbre (12) est buté à l'extrémité d'une vis d'avancement (13) pour pousser le support de pied (36) proximalement pour appliquer une charge axiale au membre et en ce que l'arbre (12) est interrompu par un transducteur de charge axiale (16) et un transducteur de moment de torsion (17).

2. Analyseur de l'articulation du genou selon la revendication 1, caractérisé en ce qu'il comprend des moyens pour contrôler continuellement des signaux EMG de la musculature du genou.

## Ansprüche

1. Gerät zur unblutigen, quantitativen Instabilitätsprüfung des Kniegelenkes am lebenden Menschen mit

Mitteln (31, 32) zur Positionierung des unter verschiedenen Winkeln gebeugten Knie- und Hüftgelenkes,

Mitteln (31, 32, 34, 41, 42, 44) zur Fixierung des Femur und der Gelenkknorren des Oberschenkelbeins,

Mitteln (36) zur Fixierung des Fusses und der Fussknöchel, die eine, auf einem rotierbaren, parallel zur Achse des Gliedes verlaufenden Schaft (12) montierte Fussplatte umfassen, wobei der Schaft (12) auf einer Anzahl von Lagern (15) ruht,

Mitteln (23) zur anterioren oder posterioren Belastung der Tibia und Mitteln (30) zur kontinuierlichen Messung der angewendeten Belastungen und Mitteln (51, 1AP) zur Messung der resultierenden anterioren oder posterioren Verschiebungen,

Mitteln (53) zur medialen oder lateralen Belastung der Tibia und Mitteln (29) zur kontinuierlichen Messung der angewendeten Belastungen und Mitteln (1ML) zur Messung der resultierenden medialen oder lateralen Verschiebungen,

Mitteln (55, 24) zur Anwendung eines Valgus- oder Varus-Momentes am Knie und Mitteln (28) zur kontinuierlichen Messung der angewendeten Momente und Mitteln (1W) zur Messung der resultierenden Valgus- oder Varus-Rotation,

Mitteln (56) zur Anwendung externer oder interner Drehmomente an der Tibia und Mitteln (26) zur kontinuierlichen Messung der angewendeten Drehmomente und Mitteln (1TT) zur Messung der resultierenden externen oder internen Tibia-Rotation,

wobei die angewendeten Belastungen über ein System von Seilscheiben (18), Seilen (24) und Gewichten (23) aufgebracht werden, dadurch gekennzeichnet, dass

Messwandler (26, 28, 29, 30) zwischen den Seilen (24) angeordnet sind,

und dass der Schaft (12) mit dem Ende einer Vorschub-Schraube (13) zusammenstösst um die Fussplatte (36) nach proximal stossen und eine axiale Belastung des Gliedes bewirken zu können und dass der Schaft (12) durch einen Messwandler (16) für axiale Lasten und einen Messwandler (17) für Drehmomente unterbrochen ist.

2. Gerät zur Instabilitätsprüfung des Kniegelenkes gemäss Anspruch 1, dadurch gekennzeichnet, dass Mittel vorgesehen sind zur kontinuierlichen Überwachung von EMG-Signalen der Kniemuskulatur.

Fig. 1

Fig. 2

KEY

AP = Anterior – Posterior
ML = Medial – Lateral
VV = Valgus – Varus
TT = Tibial Torsion

Fig. 3

EP 0 293 372 B1

Fig. 4